(19) Europäisches Patentamt
European Patent Office
Office européen des brevets

(11) EP 1 305 271 B1

(12) # EUROPEAN PATENT SPECIFICATION

(45) Date of publication and mention of the grant of the patent:
**04.03.2009 Bulletin 2009/10**

(21) Application number: **01957409.4**

(22) Date of filing: **03.08.2001**

(51) Int Cl.:
*C07C 2/32* (2006.01)

(86) International application number:
**PCT/US2001/024297**

(87) International publication number:
**WO 2002/012151 (14.02.2002 Gazette 2002/07)**

(54) **CONTINUOUS MANUFACTURING PROCESS FOR ALPHA-OLEFINS**

KONTINUIERLICHES VERFAHREN ZUR HERSTELLUNG VON ALPHA-OLEFINEN

PROCEDE DE FABRICATION CONTINUE D'ALPHA-OLEFINES

(84) Designated Contracting States:
**BE DE FR GB IT NL**

(30) Priority: **03.08.2000 US 222786 P**

(43) Date of publication of application:
**02.05.2003 Bulletin 2003/18**

(73) Proprietor: **E.I. DU PONT DE NEMOURS AND COMPANY**
**Wilmington, DE 19898 (US)**

(72) Inventor: **SCHIFFINO, Rinaldo, S.**
**Wilmington, DE 19807 (US)**

(74) Representative: **Towler, Philip Dean**
**Frank B. Dehn & Co.**
**St Bride's House**
**10 Salisbury Square**
**London**
**EC4Y 8JD (GB)**

(56) References cited:
**WO-A-00/15646**
**WO-A-98/27124**
**WO-A1-02/06192**
**WO-A1-99/02472**
**US-A- 5 955 555**

- **GIBSON V.C. ET AL: 'OLIGOMERISATION OF ETHYLENE BY BIS(IMINO)PYRIDYLIRON AND -COBALT COMPLEXES' CHEMISTRY - A EUROPEAN JOURNAL vol. 6, no. 12, 16 June 2000, VCH PUBLISHERS, US, pages 2221 - 2231**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 305 271 B1

## Description

### FIELD OF THE INVENTION

A continuous manufacturing process for α-olefins using certain iron containing ethylene oligomerization catalysts together with alkylaluminum cocatalysts, in which using a low ratio of A1: Fe in the process results in a lowered formation of undesired polyethylene waxes and polymer.

### TECHNICAL BACKGROUND

α-Olefins are important items of commerce, billions of kilograms being manufactured yearly. They are useful as monomers for (co)polymerizations and as chemical intermediates for the manufacture of many other materials, for example detergents and surfactants. Presently most α-olefins are made by the catalyzed oligomerization of ethylene by various catalysts, especially certain nickel complexes or aluminum alkyls, see for instance US4020121 and I. Kroschwitz, et al., Ed., Kirk-Othmer Encyclopedia of Chemical Technology, 4th Ed., Vol. 17, John Wiley & Sons, New York, p 839-858.

Recently, as reported in US5955555 and US6103946, it has been found that iron complexes of certain tridentate ligands are excellent catalysts for the production of α-olefins from ethylene. Among the options for using such catalysts are those in which the iron complexes are used in conjunction with a cocatalyst, particularly an alkylaluminum cocatalyst such as an alkylaluminoxane.

WO 02/06192, which is part of the state of the art by virtue of Article 54(3) EPC, discloses a manufacturing process for α-olefins using certain iron-containing ethylene oligomerization catalysts, comprising one or more liquid full reactors which are approximately at the bubble point of reacting ethylene, and optionally a final reactor which is at a lower pressure than the first reactor, both operating under specified conditions.

WO 00/15646 describes, in Example 15, a non-continuous process for the oligomerization of ethylene using certain nitrogen-containing iron complexes as catalysts and methylaluminoxane as cocatalyst. However, that process is not directed to the production of α-olefins.

It has recently been found, particularly in continuous processes using such iron complexes, that high molar ratios of Al:Fe lead to the undesirable formation of polyethylene waxes and polymers, which tend to foul the oligomerization apparatus, It has now been found that lower Al:Fe ratios diminish the formation of these undesirable polyethylenes, while not otherwise significantly deleteriously affecting the process.

### SUMMARY OF THE INVENTION

The present invention concerns a continuous process for the production of a linear α-olefin product, the process comprising the step of contacting, in a continuous reactor, process ingredients comprising an ethylene oligomerization catalyst composition, ethylene and a cocatalyst, wherein:

(a) the ethylene oligomerization catalyst composition comprises an iron complex of a compound of the formula

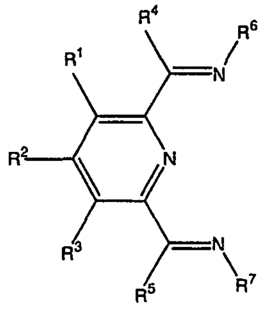

(I)

wherein:

$R^1$, $R^2$, and $R^3$ are each independently hydrogen, hydrocarbyl, substituted hydrocarbyl or an inert functional group, provided that any two of $R^1$, $R^2$ and $R^3$ vicinal to one another taken together may form a ring;
$R^4$ and $R^5$ are each independently hydrogen, hydrocarbyl, substituted hydrocarbyl or an inert functional group;

$R^6$ and $R^7$ are each independently a substituted aryl having a first ring atom bound to the imino nitrogen, provided that:

in $R^6$, a second ring atom adjacent to said first ring atom is bound to a halogen, a primary carbon group, a secondary carbon group or a tertiary carbon group; and further provided that
in $R^6$, when said second ring atom is bound to a halogen or a primary carbon group, none, one or two of the other ring atoms in $R^6$ and $R^7$ adjacent to said first ring atom are bound to a halogen or a primary carbon group, with the remainder of the ring atoms adjacent to said first ring atom being bound to a hydrogen atom; or
in $R^6$, when said second ring atom is bound to a secondary carbon group, none, one or two of the other ring atoms in $R^6$ and $R^7$ adjacent to said first ring atom are bound to a halogen, a primary carbon group or a secondary carbon group, with the remainder of the ring atoms adjacent to said first ring atom being bound to a hydrogen atom; or
in $R^6$, when said second ring atom is bound to a tertiary carbon group, none or one of the other ring atoms in $R^6$ and $R^7$ adjacent to said first ring atom are bound to a tertiary carbon group, with the remainder of the ring atoms adjacent to said first ring atom being bound to a hydrogen atom; and

(b) the cocatalyst comprises an alkyl aluminum compound;

characterized in that the molar ratio of Al in the cocatalyst to Fe in the ethylene oligomerization catalyst is from 20 to 300.

These and other features and advantages of the present invention will be more readily understood by those of ordinary skill in the art from a reading of the following detailed description. It is to be appreciated that certain features of the invention which are, for clarity, described below in the context of separate embodiments, may also be provided in combination in a single embodiment. Conversely, various features of the invention which are, for brevity, described in the context of a single embodiment, may also be provided separately or in any subcombination.

## DETAILED DESCRIPTION OF THE PREFERRED EMBODIMENTS

Herein, certain terms are used. Some of them are:

A "hydrocarbyl group" is a univalent group containing only carbon and hydrogen. As examples of hydrocarbyls may be mentioned unsubstituted alkyls, cycloalkyls and aryls. If not otherwise stated, it is preferred that hydrocarbyl groups (and alkyl groups) herein contain 1 to about 30 carbon atoms.

By "substituted hydrocarbyl" herein is meant a hydrocarbyl group that contains one or more substituent groups which are inert under the process conditions to which the compound containing these groups is subjected (e.g., an inert functional group, see below). The substituent groups also do not substantially detrimentally interfere with the oligomerization process or operation of the oligomerization catalyst system. If not otherwise stated, it is preferred that substituted hydrocarbyl groups herein contain 1 to about 30 carbon atoms. Included in the meaning of "substituted" are rings containing one or more heteroatoms, such as nitrogen, oxygen and/or sulfur, and the free valence of the substituted hydrocarbyl may be to the heteroatom. In a substituted hydrocarbyl, all of the hydrogens may be substituted, as in trifluoromethyl.

By "(inert) functional group" herein is meant a group, other than hydrocarbyl or substituted hydrocarbyl, which is inert under the process conditions to which the compound containing the group is subjected. The functional groups also do not substantially deleteriously interfere with any process described herein that the compound in which they are present may take part in. Examples of functional groups include halo (fluoro, chloro, bromo and iodo), and ether such as $-OR^{50}$ wherein $R^{50}$ is hydrocarbyl or substituted hydrocarbyl. In cases in which the functional group may be near a transition metal (Fe) atom, the functional group alone should not coordinate to the metal atom (Fe) more strongly than the groups in those compounds that are shown as coordinating to the metal atom, that is they should not displace the desired coordinating group.

By a "cocatalyst" or a "catalyst activator" is meant one or more compounds that react with a transition metal compound to form an activated catalyst species. One such catalyst activator is an "alkyl aluminum compound" which, herein, is meant a compound in which at least one alkyl group is bound to an aluminum atom. Other groups such as, for example, alkoxide, hydride and halogen may also be bound to aluminum atoms in the compound.

By a "linear $\alpha$-olefin product" is meant a composition predominantly comprising a compound (or mixture of compounds) of the formula $H(CH_2CH_2)_qCH=CH_2$ wherein q is an integer of 1 to about 18. In most cases, the linear $\alpha$-olefin product of the present process will be a mixture of compounds having differing values of q of from 1 to 18, with a minor amount of compounds having q values of more than 18. Preferably less than 50 weight percent, and more preferably less than 20 weight percent, of the product will have q values over 18. The product may further contain small amounts (preferably less than 30 weight percent, more preferably less than 10 weight percent, and especially preferably less than 2 weight percent) of other types of compounds such as alkanes, branched alkenes, dienes and/or internal olefins.

By a "primary carbon group" herein is meant a group of the formula $-CH_2$---, wherein the free valence --- is to any other atom, and the bond represented by the solid line is to a ring atom of a substituted aryl to which the primary carbon group is attached. Thus the free valence --- may be bonded to a hydrogen atom, a halogen atom, a carbon atom, an oxygen atom, a sulfur atom, etc. In other words, the free valence --- may be to hydrogen, hydrocarbyl, substituted hydrocarbyl or a functional group. Examples of primary carbon groups include $-CH_3$, $-CH_2CH(CH_3)_2$, $-CH_2Cl$, $-CH_2C_6H_5$, $-OCH_3$ and $-CH_2OCH_3$.

By a "secondary carbon group" is meant the group

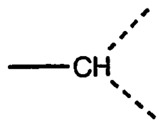

wherein the bond represented by the solid line is to a ring atom of a substituted aryl to which the secondary carbon group is attached, and both free bonds represented by the dashed lines are to an atom or atoms other than hydrogen. These atoms or groups may be the same or different. In other words the free valences represented by the dashed lines may be hydrocarbyl, substituted hydrocarbyl or inert functional groups. Examples of secondary carbon groups include $-CH(CH_3)_2$, $-CHCl_2$, $-CH(C_6H_5)_2$, cyclohexyl, $-CH(CH_3)OCH_3$, and $-CH{=}CCH_3$.

By a "tertiary carbon group" is meant a group of the formula

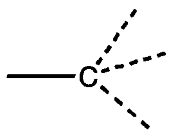

wherein the bond represented by the solid line is to a ring atom of a substituted aryl to which the tertiary carbon group is attached, and the three free bonds represented by the dashed lines are to an atom or atoms other than hydrogen. In other words, the bonds represented by the dashed lines are to hydrocarbyl, substituted hydrocarbyl or inert functional groups. Examples of tetiary carbon groups include $-C(CH_3)_3$, $-C(C_6H_5)_3$, $-CCl_3$, $-CF_3$, $-C(CH_3)_2OCH_3$, $-C{\equiv}CH$, $-C(CH_3)_2CH{=}CH_2$, aryl and substituted aryl such as phenyl and 1-adamantyl.

By "aryl" is meant a monovalent aromatic group in which the free valence is to the carbon atom of an aromatic ring. An aryl may have one or more aromatic rings which may be fused, connected by single bonds or other groups.

By "substituted aryl" is meant a monovalent aromatic group substituted as set forth in the above definition of "substituted hydrocarbyl". Similar to an aryl, a substituted aryl may have one or more aromatic rings which may be fused, connected by single bonds or other groups; however, when the substituted aryl has a heteroaromatic ring, the free valence in the substituted aryl group can be to a heteroatom (such as nitrogen) of the heteroaromatic ring instead of a carbon.

By a "first ring atom in $R^6$ and $R^7$ bound to an imino nitrogen atom" is meant the ring atom in these groups bound to an imino nitrogen shown in (I), for example

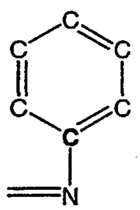

(II) or

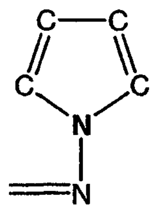

(III)

the atoms shown in the 1-position in the rings in (II) and (III) are the first ring atoms bound to an imino carbon atom (other groups which may be substituted on the aryl groups are not shown). Ring atoms adjacent to the first ring atoms are shown, for example, in (IV) and (V), where the open valencies to these adjacent atoms are shown by dashed lines (the 2,6-positions in (IV) and the 2,5-positions in (V)).

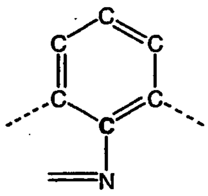 (IV) or 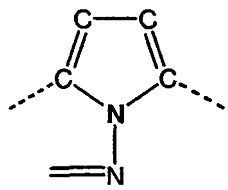 (V)

In one preferred compound (I) $R^6$ is

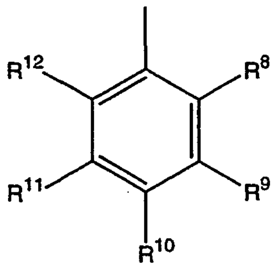

(VI)

and $R^7$ is

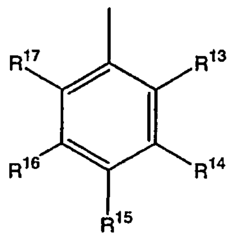

(VII)

wherein:

$R^8$ is a halogen, a primary carbon group, a secondary carbon group or a tertiary carbon group; and
$R^9$, $R^{10}$, $R^{11}$, $R^{14}$, $R^{15}$, $R^{26}$ and $R^{17}$ are each independently hydrogen, hydrocarbyl, substituted hydrocarbyl or a functional group;

provided that:

when $R^8$ is a halogen or primary carbon group none, one or two of $R^{12}$, $R^{13}$ and $R^{17}$ are a halogen or a primary carbon group, with the remainder of $R^{12}$, $R^{13}$ and $R^{17}$ being hydrogen; or
when $R^8$ is a secondary carbon group, none or one of $R^{12}$, $R^{13}$ and $R^{17}$ is a halogen, a primary carbon group or a secondary carbon group, with the remainder of $R^{12}$, $R^{13}$ and $R^{17}$ being hydrogen; or
when $R^8$ is a tertiary carbon group, none or one of $R^{12}$, $R^{13}$ and $R^{17}$ is tertiary carbon group, with the remainder of $R^{12}$, $R^{13}$ and $R^{17}$ being hydrogen;

and further provided that any two of $R^8$, $R^9$, $R^{10}$, $R^{11}$, $R^{12}$, $R^{13}$, $R^{14}$ $R^{15}$, $R^{16}$ and $R^{17}$ vicinal to one another, taken together may form a ring.
In the above formulas (VI) and (VII), $R^8$ corresponds to the second ring atom adjacent to the first ring atom bound to the imino nitrogen, and $R^{12}$, $R^{13}$ and $R^{17}$ correspond to the other ring atoms adjacent to the first ring atom.
In compounds (I) containing (VI) and (VII), it is particularly preferred that:

if $R^8$ is a primary carbon group, $R^{13}$ is a primary carbon group, and $R^{12}$ and $R^{17}$ are hydrogen; or
if $R^8$ is a secondary carbon group, $R^{13}$ is a primary carbon group or a secondary carbon group, more preferably a

secondary carbon group, and $R^{12}$ and $R^{17}$ are hydrogen; or

if $R^8$ is a tertiary carbon group (more preferably a trihalo tertiary carbon group such as a trihalomethyl), $R^{13}$ is a tertiary carbon group (more preferably a trihalotertiary group such as a trihalomethyl), and $R^{12}$ and $R^{17}$ are hydrogen; or

if $R^8$ is a halogen, $R^{13}$ is a halogen, and $R^{12}$ and $R^{17}$ are hydrogen.

In all specific preferred compounds (I) in which (VI) and (VII) appear, it is preferred that $R^1$, $R^2$ and $R^3$ are hydrogen; and/or $R^4$ and $R^5$ are methyl. It is further preferred that:

$R^9$, $R^{10}$, $R^{11}$, $R^{12}$, $R^{14}$, $R^{15}$, $R^{16}$ and $R^{17}$ are all hydrogen; $R^{13}$ is methyl; and $R^8$ is a primary carbon group, more preferably methyl; or

$R^9$, $R^{10}$, $R^{11}$, $R^{12}$, $R^{14}$, $R^{15}$, $R^{16}$ and $R^{17}$ are all hydrogen; $R^{13}$ is ethyl; and $R^8$ is a primary carbon group, more preferably ethyl; or

$R^9$, $R^{10}$, $R^{11}$, $R^{12}$, $R^{14}$, $R^{15}$, $R^{16}$ and $R^{17}$ are all hydrogen; $R^{13}$ is isopropyl; and $R^8$ is a primary carbon group, more preferably isopropyl; or

$R^9$, $R^{10}$, $R^{11}$, $R^{12}$, $R^{14}$, $R^{15}$, $R^{16}$ and $R^{17}$ are all hydrogen; $R^{13}$ is n-propyl; and $R^8$ is a primary carbon group, more preferably n-propyl; or

$R^9$, $R^{10}$, $R^{11}$, $R^{12}$ , $R^{14}$ , $R^{15}$, $R^{16}$ and $R^{17}$ are all hydrogen; $R^{13}$ is chloro; and $R^8$ is a halogen, more preferably chloro; or

$R^9$, $R^{10}$, $R^{11}$, $R^{12}$, $R^{14}$, $R^{15}$, $R^{16}$ and $R^{17}$ are all hydrogen; $R^{13}$ is trihalomethyl, more preferably trifluoromethyl; and $R^8$ is a trihalomethyl, more preferably trifluoromethyl.

In another preferred embodiment of (I), $R^6$ and $R^7$ are, respectively

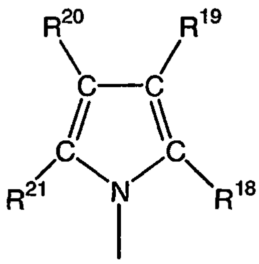

and

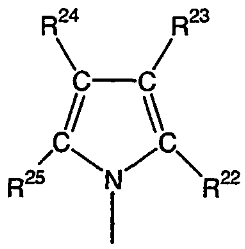

,

(VIII) (IX)

wherein:

$R^{18}$ is a halogen, a primary carbon group, a secondary carbon group or a tertiary carbon group; and

$R^{19}$, $R^{20}$, $R^{23}$ and $R^{24}$ are each independently hydrogen, hydrocarbyl, substituted hydrocarbyl or a functional group; Provided that:

when $R^{18}$ is a halogen or primary carbon group none, one or two of $R^{21}$, $R^{22}$ and $R^{25}$ are a halogen or a primary carbon group, with the remainder of $R^{21}$, $R^{22}$ and $R^{25}$ being hydrogen; or

when $R^{18}$ is a secondary carbon group, none or one of $R^{21}$, $R^{22}$ and $R^{25}$ is a halogen, a primary carbon group or a secondary carbon group, with the remainder of $R^{21}$, $R^{22}$ and $R^{25}$ being hydrogen;

when $R^{18}$ is a tertiary carbon group, none or one of $R^{21}$, $R^{22}$ and $R^{25}$ is a tertiary carbon group, with the remainder of of $R^{21}$, $R^{22}$ and $R^{25}$ being hydrogen;

and further provided that any two of $R^{18}$, $R^{19}$, $R^{20}$, $R^{21}$, $R^{22}$, $R^{23}$, $R^{24}$ and $R^{25}$ vicinal to one another, taken together may form a ring.

In the above formulas (VIII) and (IX), $R^{18}$ corresponds to the second ring atom adjacent to the first ring atom bound to the imino nitrogen, and $R^{21}$, $R^{22}$ and $R^{25}$ correspond to the other ring atoms adjacent to the first ring atom.

In compounds (I) containing (VIII) and (IX), it is particularly preferred that:

if $R^{18}$ is a primary carbon group, $R^{22}$ is a primary carbon group, and $R^{21}$ and $R^{25}$ are hydrogen; or

if $R^{18}$ is a secondary carbon group, $R^{22}$ is a primary carbon group or a secondary carbon group, more preferably a

secondary carbon group, and $R^{21}$ and $R^{25}$ are hydrogen; or

if $R^{18}$ is a tertiary carbon group (more preferably a trihalo tertiary carbon group such as a trihalomethyl), $R^{22}$ is a tertiary carbon group (more preferably a trihalotertiary group such as a trihalomethyl), and $R^{21}$ and $R^{25}$ are hydrogen; or

if $R^{18}$ is a halogen, $R^{22}$ is a halogen, and $R^{21}$ and $R^{25}$ are hydrogen.

In all specific preferred compounds (I) in which (VIII) and (IX) appear, it is preferred that $R^1$, $R^2$ and $R^3$ are hydrogen; and/or $R^4$ and $R^5$ are methyl. It is further preferred that:

$R^{19}$, $R^{20}$, $R^{21}$, $R^{23}$ and $R^{24}$ are all hydrogen; $R^{22}$ is methyl; and $R^{18}$ is a primary carbon group, more preferably methyl; or

$R^{19}$, $R^{20}$, $R^{21}$, $R^{23}$ and $R^{24}$ are all hydrogen; $R^{22}$ is ethyl; and $R^{18}$ is a primary carbon group, more preferably ethyl; or

$R^{19}$, $R^{20}$, $R^{21}$, $R^{23}$ and $R^{24}$ are all hydrogen; $R^{22}$ is isopropyl; and $R^{18}$ is a primary carbon group, more preferably isopropyl; or

$R^{19}$, $R^{20}$, $R^{21}$, $R^{23}$ and $R^{24}$ are all hydrogen; $R^{22}$ is n-propyl; and $R^{18}$ is a primary carbon group, more preferably n-propyl; or

$R^{19}$, $R^{20}$, $R^{21}$, $R^{23}$ and $R^{24}$ are all hydrogen; $R^{22}$ is chloro or bromo; and $R^{18}$ is a halogen, more preferably chloro or bromo.

Compound (I) and its iron complexes (the oligomerization catalyst) may be prepared by a variety of methods, see for instance US5955555 and US6103946, as well as US6232259 and WO00/0803

It is preferred herein to react an iron complex of (I), such as a complex of (I) with $FeCl_2$, with the cocatalyst (e.g., the alkylaluminum compound), preferably.an aluminoxane such as methylaluminoxane, to form an active ethylene oligomerization species. The molar ratio of aluminum (as alkylaluminum compound) to iron (as a complex) in the oligomerization is from 20 to 300. Within this range, a preferred lower limit is about 30 or more, and especially about 50 or more; and a more preferred upper limit about 200 or less, still more preferably about 150 or less, and especially about 100 or less. For clarity, any combination of the aforementioned upper and lower limits may be used to define a preferred range herein.

It should be noted that the above ranges refer to steady state operating conditions. Under certain circumstances, it may be beneficial to start the reaction under higher Al:Fe ratios then, in the course of the process stabilizing, lower the Al:Fe ratio to the desired steady state level. For example, the reaction could be started at above many of the upper ratio limits mentioned above, then reduced to the desired level at or above any of the lower ratio lim-, its mentioned above.

Preferred alkylaluminum compounds include one or more of $R^{51}{}_3Al$, $R^{51}AlCl_2$, $R^{51}{}_2AlCl$, and "$R^{51}AlO$" (alkylaluminoxanes), wherein $R^{51}$ is alkyl containing 1 to 25 carbon atoms, preferably 1 to 4 carbon atoms. Specific alkylaluminum compounds include methylaluminoxane (which is an oligomer with the general formula $(MeAlO)_n$), $(C_2H_5)\ _2AlCl$, $C_2H_5AlCl_2$, $(C_2H_5)_3Al$ and $((CH_3)_2CHCH_2)_3Al$. A preferred alkylaluminum compound is an aluminoxane, especially methyl aluminoxane.

The conditions for the oligomerization described in US6103946 and WO 02/06192, entitled "MANUFACTURING PROCESS FOR ALPHA-OLEFINS" (corresponding to PCT Appln. PCT/US01/22628, filed July 18, 2001), may otherwise be followed.

For example, the oligomerization reaction may be run at a wide range of temperatures generally ranging from about -100°C to about +300°C, preferably about 0°C to about 200°C, and more preferably about 20°C to about 100°C. Pressures may also vary widely, ranging from an ethylene pressure (gauge) of from about 0 kPa to about 35 MPa, more preferably from about 500 kPa to about 15 MPa.

The process may be run in gas or liquid phase, but is typically run in liquid phase, preferably using an aprotic organic liquid. The process ingredients and products may or may not be soluble in these liquids, but obviously these liquids should not prevent the oligomerization from ocurring. Suitable liquids include alkanes, alkenes, cycloalkanes, selected halogenated hydrocarbons and aromatic hydrocarbons. Specific useful liquids include hexane, toluene, benzene and the $\alpha$-olefins themselves.

The ethylene oligomerizations herein may also initially be carried out in the solid state by, for instance, supporting and active catalyst and/or aluminum compound on a substrate such as silica or alumina. Alternatively a solution of the catalyst precursor may be exposed to a support having an alkylaluminum compound on its surface. These "heterogeneous" .catalysts may be used to catalyze oligomerization in the gas phase or the liquid phase. By "gas phase" is meant that the ethylene is transported to contact with the catalyst particle while the ethylene is in the gas phase. In general, the oligomerization may be run as a continuous gas phase, solution or slurry processes.

It is particularly preferred to run the oligomerization as "essentially single phase liquid full", which means that at least 95 volume percent of the reactor volume is occupied by a liquid that is a single phase. Small amounts of the reactor volume may be taken up by gas, for example ethylene may be added to the reactor as a gas, which dissolves

rapidly under the process conditions. Nevertheless, some small amount of dissolving ethylene gas may be present. Not counted in the reactor volume is any solid resulting from fouling of the reactor. See, for example, previously mentioned WO 02/06192.

These molar ratios of Al:Fe described herein are based on the process ingredients, that is, the ingredients comprising the reactor feed; therefore, it is preferred at such low molar Al:Fe ratios to purify the process ingredients so that the alkylaluminum compounds are not "used up" reacting with moisture or other impurities.

Using the oligomerization catalysts described herein a mixture of α-olefins is obtained. A measure of the molecular weights of the olefins obtained is factor K from the Schulz-Flory theory (see for instance B. Elvers, et al., Ed. Ullmann's Encyclopedia of Industrial Chemistry, Vol. A13, VCH Verlagsgesellschaft mbH,- Weinheim, 1989, p. 243-247 and 275-276). This is defined as:

$$K = n(C_{n+2}\ \text{olefin})/n(C_n\ \text{olefin})$$

wherein $n(C_n$ olefin) is the number of moles of olefin containing n carbon atoms, and $n(C_{n+2}$ olefin) is the number of moles of olefin containing n+2 carbon atoms, or in other words the next higher oligomer of $C_n$ olefin. From this can be determined the weight (mass) fractions of the various olefins in the resulting oligomeric reaction product mixture. The K factor is preferred to be in the range of about 0.65 to about 0.8 to make the α-olefins of the most commercial interest. This factor can be varied to some extent, see for instance previously mentioned US6103946 and WO 02/06192.

## Claims

1. A continuous process for the production of a linear α-olefin product being a composition predominantly comprising a compound or mixture of compounds of the formula $H(CH_2CH_2)qCH=CH_2$ wherein q is an integer of 1 to 18, the process comprising the step of contacting, in a continuous reactor, process ingredients comprising an ethylene oligomerization catalyst composition, ethylene and a cocatalyst, wherein:

   (a) the ethylene oligomerization catalyst composition comprises an iron complex of a compound of the formula

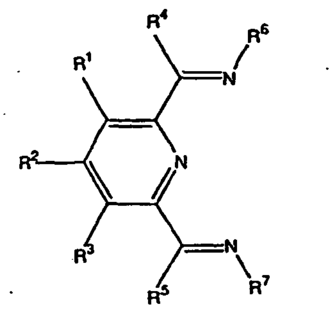

(I)

   wherein:

   $R^1$, $R^2$ and $R^3$ are each independently hydrogen, hydrocarbyl, substituted hydrocarbyl or an inert functional group, provided that any two of $R^1$, $R^2$ and $R^3$ vicinal to one another taken together may form a ring;
   $R^4$ and $R^5$ are each independently hydrogen, hydrocarbyl, substituted hydrocarbyl or an inert functional group;
   $R^6$ and $R^7$ are each independently a substituted aryl having a first ring atom bound to the imino nitrogen, provided that:

   in $R^6$, a second ring atom adjacent to said first ring atom is bound to a halogen, a primary carbon group, a secondary carbon group or a tertiary carbon group; and further provided that
   in $R^6$, when said second ring atom is bound to a halogen or a primary carbon group, none, one or two of the other ring atoms in $R^6$ and $R^7$ adjacent to said first ring atom are bound to a halogen or a primary carbon group, with the remainder of the ring atoms adjacent to said first ring atom being bound to a

hydrogen atom; or
in $R^6$, when said second ring atom is bound to a secondary carbon group, none, one or two of the other ring atoms in $R^6$ and $R^7$ adjacent to said first ring atom are bound to a halogen, a primary carbon group or a secondary carbon group, with the remainder of the ring atoms adjacent to said first ring atom being bound to a hydrogen atom; or
in $R^6$, when said second ring atom is bound to a tertiary carbon group, none or one of the other ring atoms in $R^6$ and $R^7$ adjacent to said first ring atom are bound to a tertiary carbon group, with the remainder of the ring atoms adjacent to said first ring atom being bound to a hydrogen atom; and

(b) the cocatalyst comprises an alkyl aluminum compound;

**characterized in that** the molar ratio of Al in the cocatalyst to Fe in the ethylene oligomerization catalyst is from 20 to 300.

**2.** The process of claim 1, wherein the continuous reactor is essentially single phase liquid full.

**3.** The process of claim 1, wherein said molar ratio is from 20 to 150.

**4.** The process of claim 1, wherein said molar ratio is from 30 to 200.

**Patentansprüche**

**1.** Kontinuierliches Verfahren zur Herstellung eines linearen α-Olefinprodukts, das eine Zusammensetzung ist, die überwiegend eine Verbindung oder ein Gemisch von Verbindungen mit der Formel $H(CH_2CH_2)_qCH{=}CH_2$ aufweist, wobei q eine ganze Zahl von 1 bis 18 ist; wobei das Verfahren den Schritt zum Inkontaktbringen von Prozeß-Bestandteilen, die eine Ethylen-Oligomerisationskatalysatorzusammensetzung, Ethylen und einen Cokatalysator aufweisen; in einem Durchflußreaktor aufweist, wobei:

(a) die Ethylen-Oligomerisationskatalysatorzusammensetzung einen Eisenkomplex einer Verbindung mit der Formel

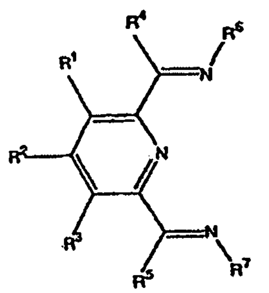

(I)

aufweist, wobei
$R^1$, $R^2$ und $R^3$ jeweils unabhängig voneinander Wasserstoff, Hydrocarbyl, substituiertes Hydrocarbyl oder eine inerte funktionelle Gruppe sind, vorausgesetzt, daß irgend zwei nachbarständige $R^1$, $R^2$ und $R^3$ zusammengenommen einen Ring bilden können;
$R^4$ und $R^5$ jeweils unabhängig voneinander Wasserstoff, Hydrocarbyl, substituiertes Hydrocarbyl oder eine inerte funktionelle Gruppe sind;
$R^6$ und $R^7$ jeweils unabhängig voneinander ein substituiertes Aryl mit einem an den Imino-Stickstoff gebundenen ersten Ringatom sind, vorausgesetzt, daß:

in $R^6$ ein dem ersten Ringatom benachbartes zweites Ringatom an ein Halogen, eine primäre Kohlenstoffgruppe, eine sekundäre Kohlenstoffgruppe oder eine tertiäre Kohlenstoffgruppe gebunden ist; und ferner vorausgesetzt, daß
wenn in $R^6$, das zweite Ringatom an ein Halogen oder eine primäre Kohlenstoffgruppe gebunden ist, keines, eines oder zwei der dem ersten Ringatom benachbarten anderen Ringatome in $R^6$ und $R^7$ an ein Halogen

oder eine primäre Kohlenstoffgruppe gebunden sind, wobei die übrigen dem ersten Ringatom benachbarten Ringatome an ein Wasserstoffatom gebunden sind; oder
wenn in $R^6$, das zweite Ringatom an eine sekundäre Kohlenstoffgruppe gebunden ist, keines, eines oder zwei der dem ersten Ringatom benachbarten anderen Ringatome in $R^6$ und $R^7$ an ein Halogen, eine primäre Kohlenstoffgruppe oder eine sekundäre Kohlenstoffgruppe gebunden sind, wobei die übrigen dem ersten Ringatom benachbarten Ringatome an ein Wasserstoffatom gebunden sind; oder
wenn in $R^6$, das zweite Ringatom an eine tertiäre Kohlenstoffgruppe gebunden ist, keines oder eines der dem ersten Ringatom benachbarten anderen Ringatome in $R^6$ und $R^7$ an eine tertiäre Kohlenstoffgruppe gebunden sind, wobei die übrigen dem ersten Ringatom benachbarten Ringatome an ein Wasserstoffatom gebunden sind; und

(b) der Cokatalysator eine Alkylaluminiumverbindung aufweist;

**dadurch gekennzeichnet, daß** das Molverhältnis von A1 in dem Cokatalysator zu Fe in dem Ethylen-Oligomerisationskatalysator 20 zu 300 beträgt.

**2.** Verfahren nach Anspruch 1, wobei der Durchflußreaktor im wesentlichen mit einphasiger Flüssigkeit gefüllt ist.

**3.** Verfahren nach Anspruch 1, wobei das Molverhältnis gleich 20 zu 150 ist.

**4.** Verfahren nach Anspruch 1, wobei das Molverhältnis gleich 30 zu 200 ist.

## Revendications

**1.** Procédé continu de production d'un produit d'α-oléfme linéaire étant une composition comprenant de manière prédominante un composé ou un mélange de composés de formule $H(CH_2CH_2)_qCH{=}CH_2$ dans laquelle q est un nombre entier prenant une valeur allant de 1 à 18, le procédé comprenant les étapes de mise en contact, dans un réacteur continu, des ingrédients du procédé comprenant une composition de catalyseur d'oligomérisation d'éthylène, de l'éthylène et un cocatalyseur, dans lequel:

(a) la composition de catalyseur d'oligomérisation d'éthylène comprend un complexe de fer d'un composé de formule

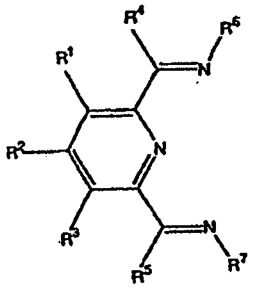

(I)

dans laquelle:

$R^1$, $R^2$ et $R^3$ sont chacun indépendamment de l'hydrogène, un groupe hydrocarbyle, hydrocarbyle substitué ou un groupe fonctionnel inerte, à condition que deux quelconques parmi $R^1$, $R^2$ et $R^3$ vicinaux l'un par rapport à l'autre pris conjointement puissent former un cycle;
$R^4$ et $R^5$ sont chacun indépendamment un atome d'hydrogène, un groupe hydrocarbyle, hydrocarbyle substitué ou un groupe fonctionnel inerte;
$R^6$ et $R^7$ sont chacun indépendamment un groupe aryle substitué ayant un premier atome cyclique lié à l'atome d'azote imino, à condition que:

dans $R^6$, un second atome cyclique adjacent audit premier atome cyclique soit lié à un atome d'halogène, un groupe carboné primaire, un groupe carboné secondaire ou un groupe carboné tertiaire; et à condition en outre que
dans $R^6$, lorsque ledit second atome cyclique est lié à un atome d'halogène ou un groupe carboné primaire, aucun, un ou deux des autres atomes du cycle dans $R^6$ et $R^7$ adjacents audit premier atome cyclique ne soit (soient) lié(s) à un atome d'halogène ou un groupe carboné primaire, le reste des atomes cycliques adjacents audit premier atome cyclique étant lié à un atome d'hydrogène; ou
dans $R^6$, lorsque ledit second atome cyclique est lié à un groupe carboné secondaire, aucun, un ou deux des autres atomes cycliques dans $R^6$ et $R^7$ adjacents audit premier atome cyclique ne soit (soient) lié(s) à un atome d'halogène, un groupe carboné primaire ou un groupe carboné secondaire, le reste des atomes cycliques adjacents audit premier atome cyclique étant lié à un atome d'hydrogène; ou
dans $R^6$, lorsque ledit second atome cyclique est lié à un groupe carboné tertiaire, aucun ou l'un des autres atomes cycliques dans $R^6$ et $R^7$ adjacents audit premier atome cyclique (ne soit) soit lié à un groupe carboné tertiaire, le reste des atomes cycliques adjacents audit premier atome cyclique étant lié à un atome d'hydrogène; et

(b) le cocatalyseur comprend un composé d'alkyl aluminium;

**caractérisé en ce que** le rapport molaire d'Al dans le cocatalyseur par rapport à Fe dans le catalyseur d'oligomérisation d'éthylène est de 20 à 300.

**2.** Procédé selon la revendication 1, dans lequel le réacteur continu est essentiellement rempli de liquide à phase unique.

**3.** Procédé selon la revendication 1, dans lequel ledit rapport molaire est de 20 à 150.

**4.** Procédé selon la revendication 1, dans lequel ledit rapport molaire est de 30 à 200.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4020121 A
- US 5955555 A
- US 6103946 A
- WO 0206192 A
- WO 0015646 A
- US 6232259 B
- WO 000803 A
- US 0122628 W


### Non-patent literature cited in the description


- Kirk-Othmer Encyclopedia of Chemical Technology. John Wiley & Sons, vol. 17, 839-858
- Ullmann's Encyclopedia of Industrial Chemistry. VCH Verlagsgesellschaft mbH, 1989, vol. A13, 243-247275-276